# EUROPEAN PATENT APPLICATION

(11) **EP 1 600 153 A1**
(43) Date of publication of application: **30.11.2005**
(21) Application number: 04012482.8
(22) Date of filing: 26.05.2004
(51) Int. Cl.: A61K 9/127, A61K 31/7076, A61K 49/10, A61K 49/18

(54) **Slow-release pharmaceutical composition allowing to monitor drug release**

(71) Applicant: Deutsches Krebsforschungszentrum Stiftung des öffentlichen Rechts, 69120 Heidelberg (DE)
(72) Inventor: Port, Rüdiger, D-69115 Heidelberg (DE); Schuster, Christian, D-69221 Dossenheim (DE); Port, Renate, D-69115 Heidelberg (DE); Bachert, Peter, D-69198 Schriesheim (DE); Zeller, Walter Jens, D-69126 Heidelberg (DE)
(74) Representative: Schüssler, Andrea

(57) **Abstract**

Described are slow-release pharmaceutical compositions allowing to monitor the course of drug release by non-invasive methods. These compositions comprise - besides a controlled release drug preparation - a detectable agent, wherein said detectable agent has substantially the same release rate as said drug.

## Description

The present invention relates to slow-release pharmaceutical compositions allowing to monitor drug release by non-invasive methods. These compositions comprise (a) a controlled release drug preparation and (b) a detectable agent, wherein said detectable agent has substantially the same release rate as said drug.

Monitoring the course of drug release of a slow (or controlled) release pharmaceutical composition is an important prerequisite for the design of the slow-release formulation and for keeping a watch on the drug level, i.e., whether the drug depot is exhausted and/or whether further applications of the drug are required for maintaining a desired drug level for successful therapy. So far, e.g., the release of a cytotoxic drug from a slow-release depot of 1-beta-D-arabinofuranosylcytosine for intrathecal therapy of cancer was monitored by determining the level of the drug in *liquor cerebrospinalis* (Kim et al., Cancer Res. 47(15) (1987), 3935-7. The localization and distribution of a drug carrier in a tissue was recorded by imaging, e.g., magnetic resonance imaging (Faas et al., Pharm. Res. 18(4) (2001), 460-6; Rubesova et al., Acad. Radiol. 9 (Suppl.2) (2002), S525-7; Lanza et al., Circulation 106(22) (2002), 2842-7) or nuclear medicine whole body gamma camera scanning (Harrington et al., Clin. Cancer Res. 7(2) (2001), 243-54). However, the imaging methods used so far only show the localization of the drug depot, however, unfortunately, not the course of drug release. Moreover, those approaches which are based on the determination of the drug level in body fluids after application of a drug depot do not allow to get information about the possible site where the drug might have migrated.

Therefore, it is the object of the present invention to provide a means which overcomes the disadvantages of the slow-release drug preparations presently used, e.g., which allows to monitor the course of release of a drug from a drug depot at the site of administration (e.g., after interstitial injection) and, thus, to decide when and/or where further drug depots have to be applied in order to maintain a therapeutic effect.

According to the present invention this is achieved by the subject matters defined in the claims. During the experiments leading to the present invention it was found that the problems of the prior art can be overcome by a slow-release pharmaceutical preparation comprising in addition to the drug a detectable agent, e.g., a contrast agent, which has substantially the same release rate as said drug. The course of the release of the drug can be indirectly monitored by any method generally available in hospitals, preferably by continuously recording the change of volume and contrast intensity of the drug depot, e.g., by imaging. Briefly, rats were subcutaneously injected with a drug depot (drug: Fludara™; slow-release compound: liposomes prepared from Phospholipon™; detectable agent: Magnevist™) and during a period of six weeks the disappearance of the drug form the site of injection was monitored *in vivo* by use of ¹⁹F-NMR-spectroscopy. At the same time, the volume and contrast intensity of the drug depot was determined by MRT imaging. Since after release from the subcutaneous depot Fludara™ is immediately absorbed and rapidly cleared the moment of disappearance from the site of injection can be regarded as corresponding to the moment of drug release. The decrease of the level of the apparent volume of the drug depot monitored by imaging paralleled the decrease of drug level at least from day 12 post injection to day 43. At day 13, 70% of the drug amounts injected were still present at the site of injection and 10% at the end of the experiment. Thus, it could be shown that by use of this combination of carrier material (liposomes), drug (Fludara™) and contrast agent (Gd-DTPA) the release of the drug can be indirectly monitored for almost the entire period of release. This period includes the terminal phase of drug release which might be relevant for a decision as regards the requirement of re-injection. For an injected volume of 1 ml, MRT-images having a slice thickness of 2 mm and a simple automatic evaluation are sufficient for monitoring the local kinetics of the contrast agent and, thus, the drug level. Thus, the release of the drug from the pharmaceutical composition of the present invention can be monitored by routine diagnosis.

### Brief description of the drawings

### Figure 1: Animal No. 1, at day 13 post injection, MRT-image (overall view) of the caudal part of the body

The region within the right flank showing increased contrast corresponds to the drug depot.

### Figure 2: Animal No. 2, transverse MRT-images showing the subcutaneously located drug depot (left column) in the right flank (1 mm sections) and the corresponding ¹⁹F-MR-spectra (right column)

The spectrum of day 1 (in the afternoon) is almost identical to the spectrum of day 1 (in the morning), thus not shown.

### Figure 3: Animal No. 2, drug level at the site of injection (left y-axis) versus time post injection (hours)

Right y-axis: relative signal intensity of the drug depot (signal intensity of the depot/signal intensity of the muscle tissue of the thigh) multiplied by the volume of the depot. All of the y-values are scaled such that the MRS-value and the MRT-value of day 13 coincide (dotted horizontal line).

### Figure 4: Animal No. 3, drug level at the site of injection (left y-axis )versus time post injection (hours)

Right y-axis: volume of the depot. All of the y-values are scaled such that the MRS-value and the MRT-value of day 13 coincide (dotted horizontal line).

### Fig. 5: Subcutaneous injection of FLAMP

Subcutaneous injection of 34 mcmol FLAMP, along with 0.5 mcmol Gd-DTPA, as a 1 ml-solution in an adult male rat, 19F MRS in vivo at the site of injection. Relative FLAMP signal (peak area of FLAMP / peak area of external NaF standard) versus time after injection.

### Fig. 6: Subcutaneous injection of Gd-DTPA

Subcutaneous injection of 0.5 mcmol Gd-DTPA (along with 34 mcmol FLAMP), as a 1 ml-solution in an adult male rat (left flank). Transversal 1H MR images (slice thickness 2 mm) from the region of the injection, taken 85 min after injection.
Images numbered in caudal-cranial direction.

Accordingly, the present invention relates to a pharmaceutical composition comprising (a) a controlled release drug preparation; and (b) a detectable agent, wherein said detectable agent has substantially the same release kinetics as the drug of (a) thereby allowing to continuously monitor the drug release.

Compounds allowing the controlled or slow-release of a drug, e.g., absorption delaying and/or diffusion inhibiting substances with high molecular weight, in an animal or human body and methods for appropriately combining said compounds with such drugs are well known to the person skilled in the art. Examples of such compounds are liposomes, nanoparticles, sugar glass microspheres (disclosed in WO 02/066005), biodegradable plastic microspheres or implants, e.g. of polylactide/glycoloide polymers (Xing et al., Biologicals 25 (1996), 57-65; Watts et al., Crit. Rev. Ther. Drug Carrier Syst. 7(3) (1990), 235-259; Kissel et al., Die gelben Hefte 23 (3) (1993), 128-134), and wafers of poly[bis(p-carboxyphenoxy)propane sebacic acid](Gliadel^{R}; Tamargo et al., Cancer Res. 53 (1993), 329-333).

The term "substantially the same physico-chemical characteristics" as used herein for characterizing the detectable compound in comparison to the controlled release drug preparation relates to properties of the agent which are responsible for the release rate. The release rates of the controlled release drug and the detectable agent have to be substantially the same, thus allowing to continuously monitoring drug release by monitoring the release of the detectable agent. In order to identify suitable combinations of drugs, carrier material and detectable agents, the person skilled in the art should generally follow a three-step procedure: 1) selection of candidate compounds based on physico-chemical properties and on known mechanisms of retention in and release from a given carrier material, 2) in-vitro testing, 3) in-vivo testing.
1) Selection of candidate compounds: The invention is applicable to drug depot preparations for local therapy and for systemic therapy. Examples of drugs in the first type of preparations would be cytostatics for the interstitial treatment of solid malignant tumors or for the prevention of local tumor recurrence, or anti-asthmatics for intrapulmonary delivery. Preparations of the second type might, for example, contain hormones for contraception or for systemic cancer treatment, psycholeptics, or vaccines. Local monitoring of drug release is preferable for the first type of depot preparations. Using a contrast agent as the detectable agent will then allow one to not only monitor the time course of drug release but also the exact site of release. This information may then be related to local effects, for example tumor shrinkage as shown by the same imaging method that is used to visualize the release of the detectable agent. MRI or X-ray contrast agents will be preferable for clinical routine applications while compounds labelled for radionuclide imaging (gamma-scintigraphy, PET, SPECT) may be useful for investigational purposes.
   For the second type of preparations, monitoring the drug at the site of administration may be of interest in the development of a depot preparation but, for clinical routine, it will be more interesting to monitor the appearance of the drug in the circulation. The detectable agent for indirect monitoring, then, should be one that is particularly easy to determine in plasma, preferably by a method that can be automated like many routine methods in laboratory medicine (Haeckel, in: Greiling et al. (eds.), Lehrbuch der Klinischen Chemie und Pathobiochemie (Stuttgart 1989), pp. 111-115). If a variety of drugs administered as depot preparations for systemic therapy could all be monitored by determining the same detectable agent in plasma, then this might greatly simplify therapeutic drug monitoring in clinical routine. The plasma concentrations of the detectable agent will usually not equal those of the drug in this case, because of different elimination rates. However, the rate of input into plasma should be the same for the drug and for an appropriately selected detectable agent. The rate of input of the detectable agent can then be estimated from non-steady-state data by pharmacokinetic modelling and, at steady state, changes of the plasma concentration will indicate changes of the rate of input of the detectable agent and, hence, of the drug.
   In general, the drug and the detectable agent should share the mechanism of retention in and release from the depot preparation. For example, liposome retain hydrophilic drugs by inclusion in the inner aqueous volume while lipophilic drugs bind to liposomal membranes. Drug release from liposome occurs by permeation through liposomal membranes, by re-partitioning of bound lipophilic drug into surrounding bulk medium, or by partial or complete disintegration of liposomal membranes. Permeability coefficients depend on molecular size, hydrophilicity and/or lipophilicity, and the degree and type of ionization (Szoka et al., Ann. Rev. Biophys. Biochem. 9 (1980), 467-508). Amphiphilic molecules permeate most easily. The actual mechanism of permeation, however, is often not completely understood (Gruner, in: Ostro (ed.), Liposomes - From Biophysics to Therapeutics (New York / Basel 1987), pp. 1-37). Thus, for liposome preparations, candidate pairs of drug and detectable agent should be similar in hydrophilicity and/or lipophilicity, molecular size and ionization. Chances for parallel release kinetics are best if both the drug and the detectable agent are released only when the liposome disintegrates. One way to achieve this is to encapsulate highly polar compounds that do not readily pass liposomal membranes. Drugs with low polarity may be modified for this purpose, e.g. by esterification with phosphoric acid, to yield a more polar prodrug which, upon release, is cleaved to generate the active form. Examples are fludarabine monophosphate (FLAMP, Examples 1, 2) which is rapidly dephosphorylated in tissue or plasma to yield the cytostatically active nucleoside, fludarabine (Hersh et al., Cancer Chemother. Pharmacol. 17 (1986), 277-280; Kuo et al., Pharmacother. 21 (2001), 528-533) and triamcinolone acetonide phosphate which has been encapsulated in liposomes for intrapulmonary administration (Gonzalez-Rothi et al., Pharm. Res. 13 (1996), 1699-1703). The monophosphate esters of the cytostatic nucleosides floxuridine (5-fluoro-2'-deoxy uridine), 5-fluoro uridine, and cytosine arabinoside could probably be used in the same way. Liposomes containing FLAMP and the extremely polar MRI contrast agent gadopentetate have proven to yield a suitable depot preparation for the purpose of the present invention (Example 2). Other candidate detectable agents for this type of preparation would be hydrophilic X-ray contrast agents as used for urography (amidotrizoate, iopromide). For a lipophilic drug that is retained by binding to liposomal membranes one might consider a lipophilic contrast agent, for example a Gd-DTPA fatty acid complex (Lucas, dissertation, Berlin 2001: www.diss.fuberlin/2001/190/). However, one prerequisite for the concept of this invention to work is that the detectable agent is rapidly eliminated from the site of the depot preparation upon release (see below, section "In vivo testing"). This condition may not be fulfilled by a lipophilic contrast agent as it may locally bind to tissue components so that it may be preferable to stay with a hydrophilic contrast agent and to convert the lipophilic drug into a hydrophilic prodrug.
   Drugs are retained in microcapsules by inclusion in an inner liquid reservoir, and are retained in microparticles or rod or wafer implants in the form of a solid solution or solid dispersion. Drug release proceeds by diffusion through the solid phase, by diffusion in water that slowly penetrates the polymer matrix producing pores and furrows ("pore diffusion"), or by erosion of the polymer matrix (Kissel et al., in: Müller BW (ed.), Controlled Drug Delivery (Stuttgart 1987), pp. 103-132; Kissel et al., Deutsche Apotheker Zeitung 133 (23) (1993), 2079-2082; Mank et al., Pharmazie 46 (1991), 9-18). Diffusivity in polymers is largely a function of molecular size (Longer et al., in: Gennaro AR (ed.), Remington's Pharmaceutical Sciences (18 edition, Easton/PA 1990, pp. 1676-1693). Thus, candidate pairs of drug and detectable agent should be similar in aqueous solubility and in molecular size, and chances for simultaneous release will again be best if the erosion of the polymer matrix is the predominant determinant of drug release rather than diffusion through the solid phase or pore diffusion.
2) In vitro testing: The actual kinetics of the release from liposomes, microspheres or solid implants cannot be predicted with sufficient accuracy from physico-chemical information alone and must be determined experimentally for both the drug and the detectable agent. In vitro tests should precede experiments in vivo for an early exclusion of non-suitable preparations and an optimisation of remaining candidate preparations. A suitable in vitro experiment is the incubation of a liposome suspension in buffer or plasma at 37 in a rotating reagent vial mixer for specified time periods, followed by centrifugation and HPLC determination of the contents of drug and detectable agent in extracts of supernatant and pellet. Release from biodegradable polymers may be determined by incubation without agitation, shaking for a few seconds at defined sampling time points, sampling the release medium with replacement, and HPLC determination of drug and detectable agent contents in the removed medium.
3) In vivo testing: Release kinetics in vivo may differ from those in vitro. Examples are an increase of liposomal membrane permeability by the presence of exogenous proteins (Szoka et al., Ann. Rev. Biophys. Biochem. 9 (1980), 467-508)or the enzymatic degradation of drug-carrying polymers (Kissel et al., Deutsche Apotheker Zeitung 133 (23) (1993), 2079-2082). Thus, the development of a suitable depot preparation requires to also determine the release kinetics of the drug and the detectable agent in vivo. Once it has been established that they run in parallel, or at least that there is a predictable constant relationship between both, then it will be sufficient to monitor the detectable agent - which is the projected benefit from the present invention.

If the drug depot preparation is developed for local effectiveness, then the release kinetics of both the drug and the detectable agent should be determined locally. The most convenient way of local drug monitoring is in vivo MRS. It requires the drug molecules to move freely in a solution, as in the aqueous phase of liposomes or in the liquid core of microcapsules. Sensitivity is best with fluorine-containing drugs (Port et al., Inv New Drugs 21 (2003), 157-168) (cf. Example 2). Drug molecules without a fluorine atom may be detectable by 1H MRS, or may be labelled with (nonradioactive) 13C. The notorious sensitivity problem of in vivo MRS is greatly alleviated by measuring at the site of an interstitial drug administration, as opposed to the more common measurements in tissues after systemic administration (Port et al., Cancer Chemother. Pharmacol. 44 (1999), 65-73).

The amount of drug, as determined locally by MRS in vivo, reflects the kinetics of release from the depot preparation only if the drug becomes rapidly undetectable upon release. If the drug is monitored by MRS in vivo, then this condition is fulfilled if, upon release, bound to macromolecules or is eliminated via the circulation. If metabolites are formed locally, then they must have resonance frequencies different from that of the parent compound, or must themselves rapidly become undetectable in order to not compromise the interpretation of the signal from the parent drug. The MRS signals that will locally be generated by the drug upon release from the depot can be simulated by injecting the drug as a solution. This has been done for FLAMP showing that signals from the drug itself or from metabolites that cannot be distinguished by their resonance frequency disappear within a few hours which is sufficiently rapid if the release of drug from the depot goes on for weeks (Example 3).

The drug will not be detectable by MRS in vivo if it is bound to liposomal membranes or exists in a solid form in microspheres or solid implants. In animal experiments, drug concentrations may then be measured invasively, by taking tissue samples from the administration site. A substitute for measuring local drug concentrations, even for drugs that are applied for local therapy, may be to monitor drug concentrations in plasma and to estimate the rate of release from the depot preparation by pharmacokinetic modelling.

Monitoring the detectable agent at the site of administration will be no problem because, for local monitoring, it has been chosen to be easily detectable by an imaging method like MRI or CT. If the detectable agent is an MRI contrast agent and the drug is monitored by in vivo MRS, then both can be measured in one session in the same MR tomograph. This procedure is especially suited for repeated examinations in animals or humans as it is non-invasive and even involves no radiation.

The detectable agent should be one that is rapidly eliminated from the site of administration upon release from the depot preparation. The released agent must not interfere with the imaging of the agent that is still remaining in the depot preparation. Local clearance of the detectable agent may proceed by passage through capillary walls into the circulation or by lymphatic drainage. Local concentration may also drop by distribution in a large tissue space. The local detectability of free Gd-DTPA has been tested by MR imaging after a subcutaneous injection of Gd-DTPA solution (Example 3). Almost no contrast enhancement was left at the site of administration at one and a half hours after the injection so that, for Gd-DTPA, local elimination (or distribution) is sufficiently rapid if the mean time of release from a drug depot is in the order of weeks. The same can be expected for low-molecular weight hydrophilic X-ray contrast agents with low protein binding, e.g. amidotrizoate or iopromide.

Local clearance may not be sufficiently rapid with highly lipophilic contrast agents as they may bind to cell membranes, and will most likely be too slow with macromolecular contrast agents like albumin-Gd-DTPA or Gd24-cascade polymer (Daldrup et al., Radiologe 37 (1997), 733-740; Daldrup-Link Eur. Radiol. 13 (2003), 354-365).

If the intended clinical use of the drug is for systemic therapy, as in the case of hormones for contraception or for cancer treatment, or of psycholeptics for the treatment of schizophrenia, then the kinetics of the drug and the detectable agent may be monitored at the site of administration but should also be monitored in plasma. For experimental testing, drug concentrations in plasma can be determined by established methods like HPLC or immunoassays (Oellerich, in: Greiling et al. (eds.), Lehrbuch der Klinischen Chemie und Pathobiochemie (Stuttgart 1989), pp. 1096-1114).

The measurement method for the detectable agent should in this case be one that can be automated like many of the routine methods in laboratory medicine (Haeckel, in: Greiling et al. (eds.), Lehrbuch der Klinischen Chemie und Pathobiochemie (Stuttgart 1989), pp. 111-115).

For administration, the compounds of the pharmaceutical composition of the invention can be combined with further suitable pharmaceutical carriers. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the suitable compositions may be effected by different ways, e.g. by intravenous, intraperitoneal, subcutaneous, intramuscular, topical or intradermal administration. The route of administration, of course, depends on the particular nature of the disease and, e.g., the location of a tumor as well as the kind of compound contained in the pharmaceutical composition. The dosage regimen will be determined by the attending physician and other clinical factors. As is well known in the medical arts, dosages for any one patient depend on many factors, including the patient's size, body surface area, age, sex, the particular compound to be administered, time and route of administration, the kind and stage of the disease, general health and other drugs being administered concurrently.

In a preferred embodiment, the detectable agent of the pharmaceutical composition of the present invention is an agent that is detectable upon imaging, e.g., magnetic resonance imaging (MRI), X-ray computer tomography (CT), positron emission tomography (PET), single-photon emission computer tomography (SPECT) or gamma-scintigraphy.

In a more preferred embodiment of the present invention, said detectable agent is a paramagnetic ion, radioactive ion or a fluorogenic ion. Specifically, the detectable (or diagnostic) agents utilized in embodiments of the invention include: chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III), erbium (III), iodine¹²³, technetium^{99m}, indium^{111,} rhenium¹⁸⁸, rhenium¹⁸⁶, copper⁶⁷, iodine¹³¹, yttrium⁹⁰, iodine¹²⁵, astatine²¹¹, and gallium⁶⁷.

In an even more preferred embodiment of the present invention, said detectable agent is a contrast agent, preferably a contrast agent useful for MRI (or MRT) or CT. Particularly preferred is gadolinium-DPTA (Magnevist™).

Any drug that is useful for therapy and which can be formulated in a controlled release composition is useful for the purposes of the present invention. Preferred examples of such drugs are anticancer drugs, preferably Fludarabin-5'-monophosphate (Fludara™), a hormone or an antipsychotic drug.

In one of the most preferred embodiments of the pharmaceutical composition of the present invention said anticancer drug is Fludarabin-5'-monophosphate (Fludara™) and said contrast agent is gadolinium-DPTA (Magnevist™).

Preferred compounds for the preparation of controlled release formulations are microparticles or liposomes with liposomes prepared from Phospholipon™ 90H being particularly preferred.

Preferably, imaging is used for monitoring drug release. Suitable imaging methods are well known to the person skilled in the art and comprise MRT, PET, CT, SPECT, gamma-scintigraphy.

The present invention also relates to various therapeutic uses of the compounds of the present invention. It can be expected that a pharmaceutical preparation of the present invention is useful for relapse prophylaxis after surgical removement of a brain tumor by injecting multiple depots into the edges of the resection cavity. For this approach anticancer drugs like fludarabinmonophosphate might be particularly advantageous since due its extremely high polarity it is not readily eliminated through the capillary walls in brain ("blood-brain barrier") and might be preferably accumulated in malign glioma cells since these cells exhibit enhanced activity of membrane bound 5'-nucleotidase (Amano et al., Neuropathol. 59 (1983), 145-149) which enables the import into the cells by cleavage of the phosphate residue. By use of MRT-imaging it can be determined when and/or where further applications of the drug are necessary.

Thus, a preferred use of a pharmaceutical composition of the present invention is relapse prophylaxis of a tumor, in particular relapse prophylaxis of gliomas after surgical removal. A preferred use is also the interstitial treatment of mainifest tumors, such as gliomas and other tumors where interstitial drug administration has shown promising results: primary and secondary tumors of the liver (Garcea et al., Eur. J. Cancer 39 (2003), 2150-2164), prostate cancer (Le Pivert et al., Technol. Cancer Res. Treatment 3 (2004), 135-142), basal cell carcinoma (Miller et al., J. Am. Acad. Dermatol. 36 (1997), 72-77) and hypopharyngeal squamous cell carcinoma (Chung et al., Clin. Laser Med. Surg. 21 (2003), 23-27). Another preferred use is the treatment of lung diseases by intrapulmonary administration of liposome preparations containing, for example, anti-asthmatic or anti-infective agents (Schreier in: Lasic DD et al., (eds.), Medical Applications in Liposomes (Amsterdam etc. 1998), pp. 473-492).

Further uses of the pharmaceutical composition of the present invention comprise the monitoring of drug release from interstitially applied depots containing drugs for systemic therapy: hormones for contraception or for cancer treatment (Hoffmann et al., in: Zeller/zur Hausen, Onkologie - Grundlagen, Diagnostik, Therapie, Entwicklungen (17. Erganzungslieferung, Landsberg 2004), pp. IV-5, 1-71) or psycholeptics. In this case, the detectable agent may be either a contrast agent for imaging at the site of administration or a compound which, after release from the depot, is particulary easy to determine in plasma, as decribed above. It can be also expected that drugs (e.g., cytosine-arabinoside, 5'-fluoro-2'-deoxyuridine) can be esterized with phosphoric acid in such a way that in the tissue after cleavage of the ester bond the active drug is released and again available. Such phosphoric esters can be used for the slow-release preparations of the present invention, preferably in combination with liposomes and/or Gd-DPTA.

The present invention is explained by the following examples.

### Example 1

### General methods

### (A) Materials

Phospholipon™ (Nattermann, Phospholipid GmbH, Germany, Cologne), Fludarabin-5'-monophosphate (Fludara™, Schering, Germany, Berlin) and gadolinium-DPTA (Magnevist™, Schering).

### (B) Preparation of a slow-release pharmaceutical composition containing gadolinium-DPTA (Magnevist™, Schering)

2 ml aqua bidest., 1,7 µl Magnevist™ and 200 mg Phospholipon™ 90H were added to a bottle for injection of Fludara™. The mixture was mixed by hand for 20 min. at room temperature. Then, the mixture was subjected to 6 cycles of vortexing for 2 min. and placing in a water bath at 64°C for 3 min.. Subsequently, the mixture was subjected to 6 cycles of holding in liquid nitrogen for 3 min. and placing in a water bath at 64°C for 6 min. Finally, the mixture was washed for four times with each of 10 ml phosphate buffer. After each washing step, the mixture was centrifuged for 15 min. at 10,000 x g. A colorless preparation having a consistency of a paste was obtained which was transferred to a syringe.

### Example 2

### Animal testing: Depot preparation

### (A) Animals/treatment

Adult male Wistar rats were bred in twos in isolators and received Altromin™ and water ad *libitum.* At day 1, in the morning rats were subcutaneously injected once with 1 ml of the pharmaceutical composition described in Example 1 into the right flank ("Luer"-canulla No.12 with screw thread). Injections and NMR-measurements were carried with rats which had been previously subjected to inhalation anesthesia (77% N₂, 22% O₂ and 1 - 1,2% Isofluran™) .

### (B) Experimental setting

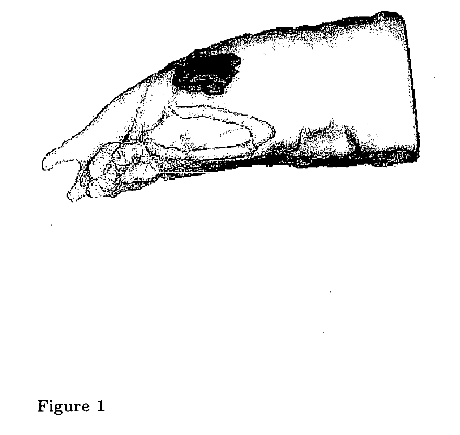

Results are shown in Figures 1 to 4. At day 1, in the morning (after the formulation had been injected) and in the afternoon, respectively, as well as at days 4, 13, 22, 29, 36 and 43 the following NMR-measurements were carried out: The amount of Fludarabin monophosphate (FLAMP) at the site of injection was determined by ¹⁹F-MRS and the volume and the contrast intensity of the drug depot was measured by MRT. The results are shown in Figure 1.

### (C) NMR-methods

A 1,5-T whole body tomograph(Magnetom Vision Plus, Siemens AG, Erlangen, Germany) was used for the NMR-measurements. For both, the ¹⁹F-MRS and the ¹H-imaging a volume resonator which had been constructed by the DKFZ (resonance frequency: 63,6 MHz (¹H) , 59,9 MHz (¹⁹F) with an inner diameter of 8.4 cm was used which served as transmitting- and receiving coil.

After the rats had been positioned (subcutaneous liposome depot in the middle of the resonator) the volume resonator was manually tuned to the ¹H resonance frequency. After "Shim" (optimization of field homogeneity) by use of the ¹H-tissue water resonance line and subsequent tuning of the coil to the ¹⁹F resonance frequency the ¹⁹F spectra were recorded. The reading sequence comprised a simple non-layer-specific pulsed excitation with subsequent recording of 1024 readings in 51.2 ms corresponding to the fast T₂-decay of the FID. The recording period of TA = 33:21 min. resulted from a repetition time TR = 100 ms and a number of NEX = 20000 excitations.

In order to get a reference for the chemical shift and the signal intensity of FLAMP a 0.5 ml reference solution containing 400 mM NaF was applied to the middle of the underside of the volume resonator with the resonance of this solution being at about -40 ppm (with reference to TFA). The follow-up editing of the spectra was carried out by use of the software LUISE which is implemented to the tomograph. All of the FIDs were expanded to 4096 data points by zero-filling and multiplied by a Gauß' function prior to Fourier transformation and phase correction. After correction of the basal line by use of a spline function the spectra were automatically fitted (on the assumption that the resonance lines correspond to a Gauß' function). The relation of the integrals of the resonances of FLAMP to NaF served as a reference value for the individual readings.
¹H-imaging with high resolution was carried with a T₁-weighted 3D-gradient-echo-method (3D-FLASH) with lipid saturation (parameters used for measurements: TR = 45 ms; TE = 11 ms; α = 30; FOV = 50 mm; matrix-size = 128 x 128; period of acquisition for the individual readings: 15,36 ms). Multiple transverse layer images of the liposome depots were recorded with layers having an actual thickness of 2, 1- and 0,4 mm, respectively. Resolution within layers was 0.39 x 0.39 mm² in all cases. For images of layers of 2 mm and 1 mm, respectively, the number of acquisitions was NEX = 1, images of layers of 0,4 mm were recorded with NEX = 4 in order to improve the signal-to-noise ratio (SNR), resulting (according to the overall thickness of the imaged volume, mostly 36 mm) in recording periods of about 2 min. (2 mm), about 4 min. (1 mm) and about 35 min. (0,4 mm actual depth of the layers).

Images were segmented with the publicly available software ImageJ for determination of the volume and signal intensity of the liposome depot by setting a minimum threshold for the signal intensity of the liposome depot using a 3D-histograph. The relative signal of the liposome depot(in relation to the reference signal of muscle tissue of the thigh) multiplied with the volume measured was used as reference value for the individual measurements.

### (D) Results and discussion

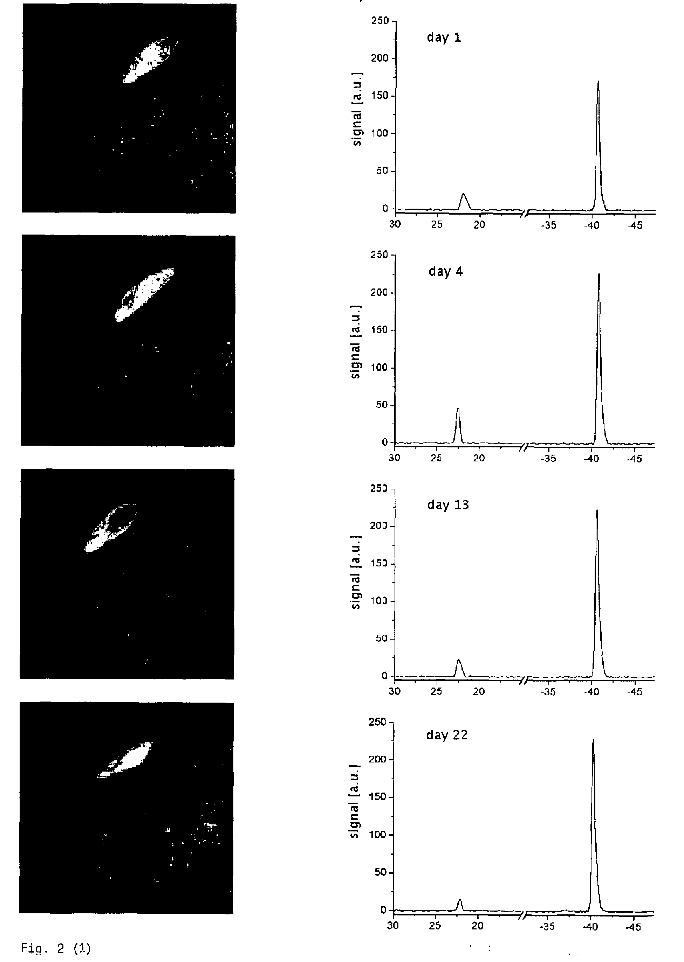

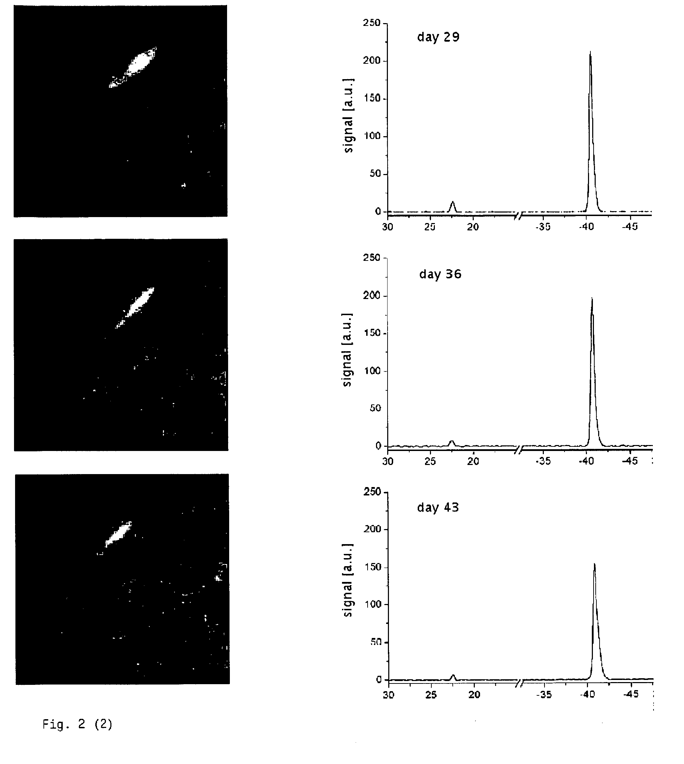

During the whole of six weeks the ¹⁹F-spectra indicated significant amounts of FLAMP at the site of injection (Figure 2, right column). The amount of FLAMP is in proportion to the area under the FLAMP peak. The shape of the FLAMP peak (height, breadth) depends on the corresponding Shim-result but does not depend on the amount. In addition to FLAMP, Isofluran™ could always be detected and occasionally low amounts of trifluoro acetic acid (metabolite of Isofluran™) could be detected. About one week after injection the amount of FLAMP at the site of injection started to decrease continuously. For the entire period of the experiment the drug depot was visible on the MRT-images as an area with increased contrast (Figure 1, Figure 2, left column). On the images at day 1 and day 4 larger areas which were adjacent to each other showed complete signal extinction. Since the reason for this phenomenon was not known, for evaluation only images recorded one week after injection (or later) were used. One week after injection (and later) only isolated areas showing signal extinction were observed. The diameter of these areas (resembling small bubbles of air) was about 1 to 2 mm.

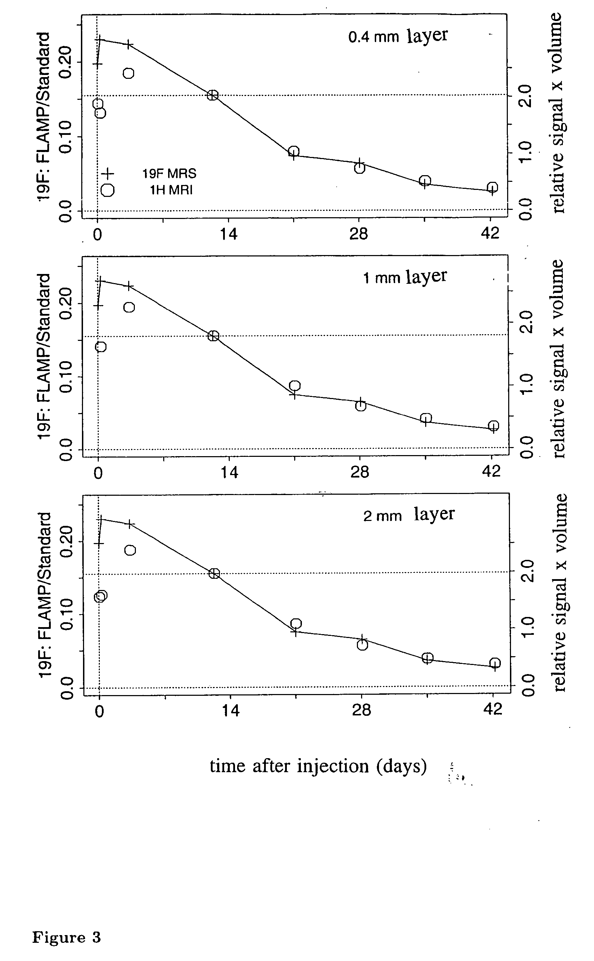

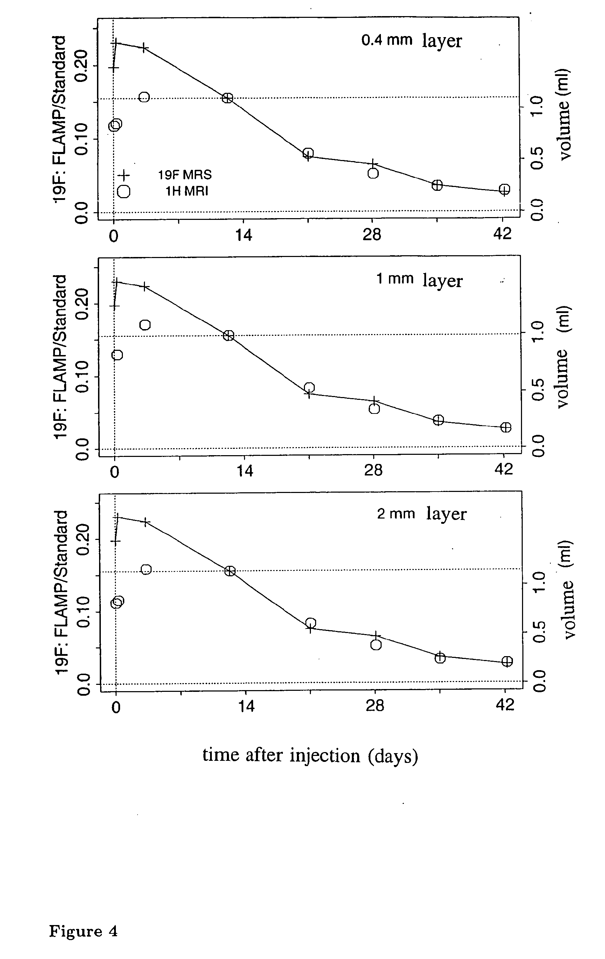

Two weeks after injection (at the latest) the decrease of the amounts of the drug (FLAMP) at the site of injection and the decrease of the amounts of contrast agent started to run in parallel (Figures 3 and 4; the first NMR examination following those on days 1 and 4 was that on day 13). For the determination of the amounts of contrast agent two parameters were used: (a) The contrast intensity of the depot/reference tissue (muscle tissue of the thigh)-ratio multiplied by the volume of the depot (Figure 3), or (b) just the volume of the depot (Figure 4). Both MRT-parameters yielded almost identical results as regards the decrease of the amounts of drug in the course of time until the end of the experiment (after six weeks; Figures 3 and 4.). For the images with layer depths of 0.4, 1 and 2 mm, respectively, the quality of the correspondence between the course of MRS and MRT was almost identical (Figures 3 and 4).

After subcutaneous application FLAMP is immediately absorbed such that the course of the change of plasma level corresponds to the change after intravenous application (Kuo et al., Pharmacother. 21 (2001), 528-533). In other words, the course of time of the decrease of drug level at the site of injection is equivalent to the course of release from the depot. The decrease of size of the depot in the tissue as determined by MRT imaging runs parallel. Accordingly, the release of the drug can be indirectly monitored by MRT imaging (at least during the period from about one week to about six weeks after subcutaneous injection). During this period the amounts of drug available for release drops from 70 to 12% of the amounts which had been initially injected.

Thus, by use of a preparation of the present invention the release of the drug from the tissue can be indirectly monitored by MRT imaging. Most importantly, such medical examinations are non-invasive. Due to its very short recording time for 2 mm images (2 min. for a tissue volume of 5 x 5 x 4 cm³) this method can be used for clinical routine examinations. The MRT images are quantitatively meaningful and can be interpreted for almost the entire period of drug release including the terminal phase of release. Thus, after interstitial injection of one or more drug depots on the basis of MRT measurements it can be decided (a) when and/or (b), if necessary, where further injections have to be applied in order to maintain the desired therapeutic effect.

### Example 3

### Animal testing: Solution

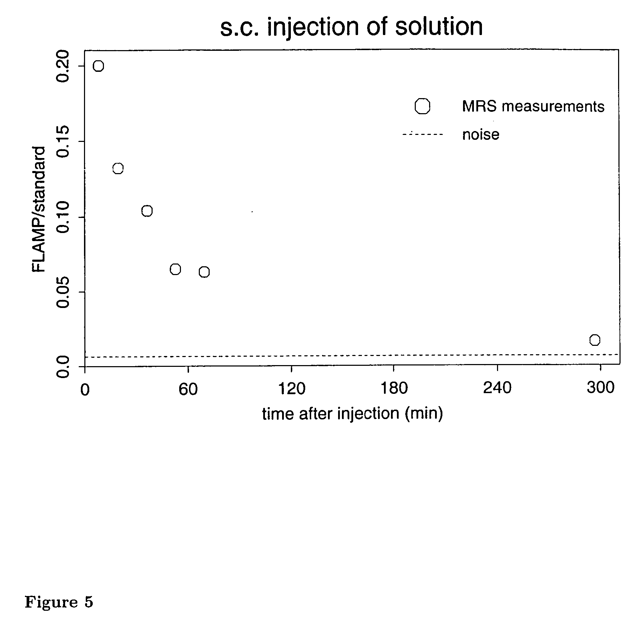

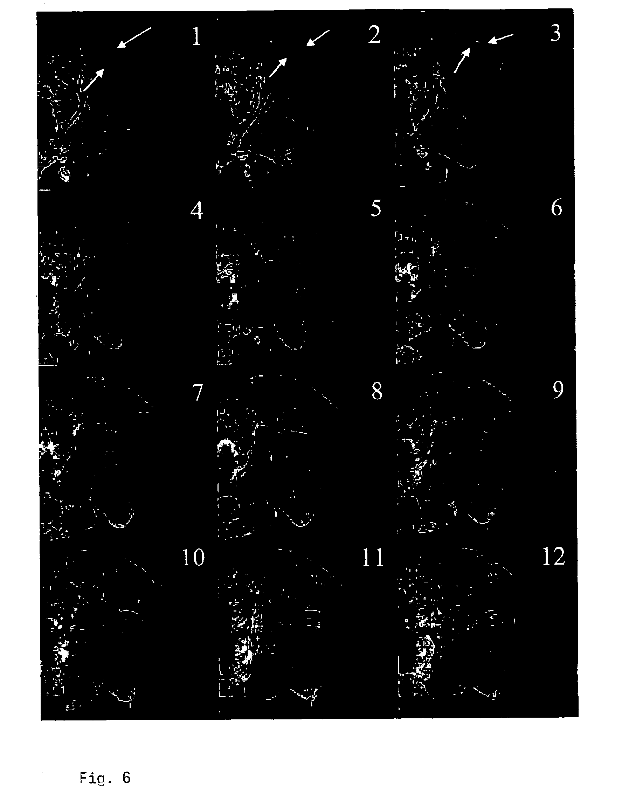

A solution of the drug and the contrast agent was injected subcutaneously, followed by local MRS and MRI monitoring as in Example 2, in order to test whether either agent might be locally detectable even after release from the depot preparation. The injection volume (1 ml) and the amount of drug (FLAMP, 34 mcmoles) were the same as in Example 2 while the amount of contrast agent was doubled (Gd-DTPA, 0.5 mcmol). The 19F MRS signal from the drug at 8 min after the injection was about the same as on day 1 after injecting the depot preparation but dropped by more than 90 within 5 hours, and was no longer detectable after 24 hours (Figure 5).
1H-images were taken from 24 adjacent 2 mm layers in the region of the injection at 85 min after the injection and only a faint diffuse contrast enhancement was detectable in three adjacent layers, in an area of about 212 mm (Figure 6). The amounts of FLAMP and of Gd-DTPA that were released from the depot preparation on any one day in the experiment of Example 2 were much less than what was injected all at once in this control experiment so that it can be concluded that the signals from the drug and the contrast agent, as monitored locally over six weeks after the injection of the depot preparation in Example 2, did not contain appreciable contributions from released drug or contrast agent.

## Claims

1. A pharmaceutical composition comprising:
(a) a controlled release drug preparation; and
(b) a detectable agent, wherein said detectable agent has substantially the same release rate as the drug of (a) thereby allowing to continuously monitor the drug release.

2. The pharmaceutical composition of claim 1, wherein said detectable agent is a contrast agent.

3. The pharmaceutical composition of claim 1 or 2, wherein said contrast agent is an agent useful for MRI.

4. The pharmaceutical composition of claim 3, wherein said contrast agent is gadolinium-DPTA (Magnevist™).

5. The pharmaceutical composition of any one of claims 1 to 4, wherein said drug is an anticancer drug, a hormone or an antipsychotic drug.

6. The pharmaceutical composition of claim 5, wherein said anticancer drug is Fludarabin-5'-dihydrogen phosphate (Fludara™) .

7. The pharmaceutical composition of any one of claims 4 to 6, wherein said anticancer drug is Fludarabin-5'-monophosphate (Fludara™) and said contrast agent is gadolinium-DPTA (Magnevist™) .

8. The pharmaceutical composition of any one of claims 1 to 7, wherein said drug is contained in liposomes, micro- or nanoparticles, or biodegradable polymer implants.

9. The pharmaceutical composition of claim 8, wherein said liposomes are Phospholipon™ 90H.

10. The pharmaceutical composition of any one claims 1 to 9, wherein imaging or plasma level monitoring of a detectable agent other than the drug itself is used for monitoring drug release.

11. Use of the compounds as defined in any one of claims 1 to 10 for the preparation of a pharmaceutical composition for relapse prophylaxis of a tumor.

12. Use according to claim 11, wherein said tumor is a glioma.
